# DEMANDE DE BREVET EUROPEEN

(11) **EP 0 596 402 A1**
(43) Date de publication de la demande: **11.05.1994**
(21) Numéro de dépôt: 93117449.4
(22) Date de dépôt: 28.10.1993
(51) Int. Cl.: F21L 11/00, A61B 1/24

(54) **Dispositif portatif d'éclairage**

(30) Priorité: 03.11.1992 CH 3411/92
(71) Demandeur: SURGICAL MEDICAL CREATIONS (SMC) - CREATIONS CHIRURGICO-MEDICALES LIMITED, Cork (IE)
(72) Inventeur: Johannot, Anne-Marie, CH-1175 Lavigny (CH)
(74) Mandataire: KIRKER & Cie S.A.

(57) **Abrégé**

Le dispositif portatif d'éclairage comprend un corps (1) de forme allongée, prolongé par une tige (5). Cette tige présente à son extrémité libre une tête (6) munie d'une source lumineuse (10) alimentée par des piles (2) disposées dans le corps (1). Elle est articulée par une rotule (7) de telle sorte qu'elle puisse tourner autour de son axe longitudinal et être inclinée par rapport à cet axe.

Un miroir (15) porté par un bras (16) peut être vissé de façon amovible dans l'un des deux filetages (14) situés de part et d'autre de la source lumineuse (10).

Ce dispositif autonome d'éclairage efficace permet la transillumination ainsi que l'observation directe. Le miroir permet les observations indirectes droite ou gauche.

## Description

L'invention concerne un dispositif portatif d'éclairage, comprenant un corps de forme allongée, prolongé par une tige, cette tige présentant à son extrémité libre une tête munie d'une source lumineuse.

Dans le domaine de la médecine, il est connu d'utiliser des dispositifs d'éclairage pour l'observation clinique bucco-dentaire et le diagnostic par diaphanoscopie ou transillumination. Les dispositifs d'éclairage connus sont souvent reliés à un générateur par un fil et ils sont d'un emploi peu pratique en raison de leur forme.

L'invention a pour but de fournir un dispositif portatif et autonome d'éclairage efficace, permettant l'observation directe, indirecte droite ou gauche et la transillumination.

L'invention a pour objet un dispositif portatif d'éclairage, comprenant un corps de forme allongée, prolongé par une tige, cette tige présentant à son extrémité libre une tête munie d'une source lumineuse, caractérisé en ce que la tige est articulée a l'une des extrémités du corps, et en ce que la tête comporte une source lumineuse décalée par rapport à l'axe de la tige et au moins un miroir monté de façon amovible d'une part ou d'autre part de la source lumineuse.

La tige est articulée à l'extrémité du corps de préférence par une rotule, de telle sorte qu'elle puisse tourner autour de son axe longitudinal et être inclinée par rapport à cet axe.

La tige articulée peut être inclinée jusqu'à un angle d'environ 22 _{°} par rapport à l'axe longitudinal du corps.

La tige articulée peut tourner autour de son axe longitudinal sur 360°.

La source lumineuse est de préférence une ampoule halogène, alimentée par des piles disposées dans ledit corps.

La tige est creuse et est parcourue par un conducteur électrique reliant l'un des contacts de la source lumineuse à l'un des pôles de la pile, l'autre contact de la source lumineuse étant relié à l'autre pôle de la pile par le corps, la tige et la tête.

Dans une forme d'exécution de l'invention, le miroir est porté par un bras situé à 90 _{°} l'un par rapport à son emplacement symétrique, de telle sorte que la source lumineuse soit située sur la bissectrice des axes des bras pouvant porter le miroir.

Le bras est vissé dans l'un des deux filetages pratiqués dans la tête.

Le miroir est de préférence perpendiculaire au bras qui le porte. Le miroir est plan ou concave.

Les dessins annexés représentent à titre d'exemple, deux formes d'exécution de l'invention.

La figure 1 représente une forme d'exécution du dispositif selon l'invention.
La figure 2 représente la tête d'une variante de cette forme d'exécution comprenant le miroir.
La figure 3 représente la tête de la figure 2 vu selon l'axe A.

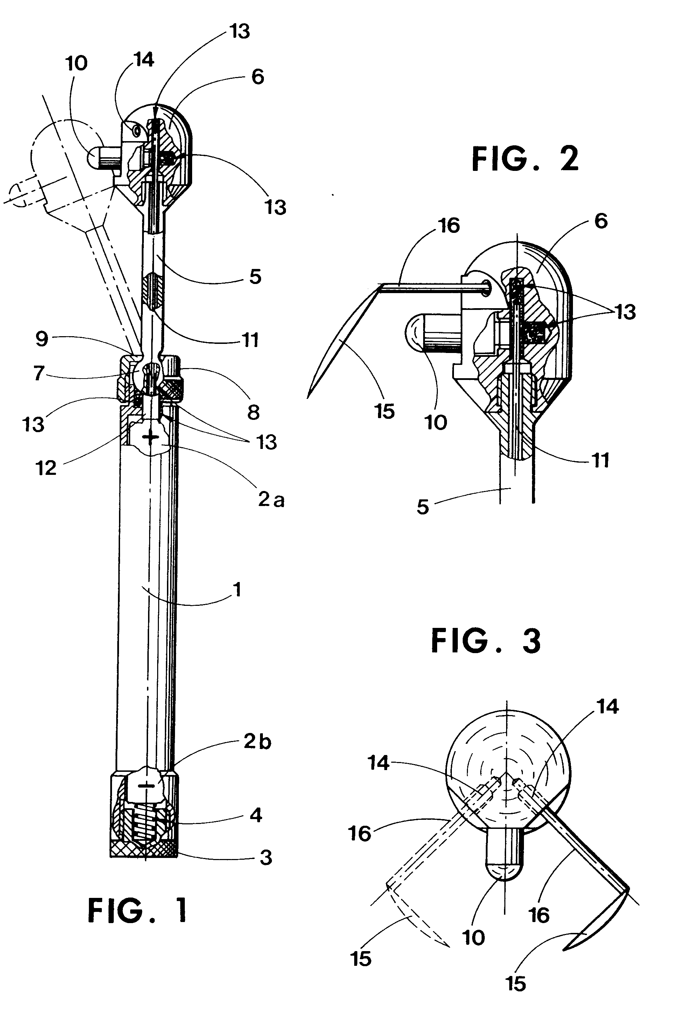

Le dispositif représenté sur la figure 1 comporte un corps 1 de forme allongée contenant deux piles 2a, 2b de 1,5 Volts.

Il comporte à son extrémité postérieure un bouchon 3 dévissable pour introduire ou retirer les piles 2 du corps 1.

Ce bouchon 3 comporte un ressort 4 destiné à maintenir les piles 2 en place et à établir le contact électrique.

L'autre extrémité du corps 1 est prolongée par une tige 5 articulée portant une tête 6 d'éclairage.

La tige 5 présente une extrémité en forme de rotule 7 qui est montée à l'intérieur d'une cage formée par les parois internes d'un écrou 8. Cet écrou 8 est vissé sur un pas de vis que présente l'extrémité antérieure du corps 1.

L'écrou 8 est percé d'une ouverture 9 permettant à la tige 5 de pivoter autour de son axe longitudinal sur 360 d'une part, et d'être inclinée par rapport à l'axe longitudinal du corps 1 jusqu'à un angle d'environ 22°.

La tête 6 est munie d'une source lumineuse 10 constituée d'une ampoule halogène miniature disposée perpendiculairement à l'axe longitudinal de la tige 5, alimentée électriquement par les piles 2.

La tige 5 et sa partie en forme de rotule 7 sont percées d'un conduit pour le passage d'un fil 11 permettant de relier électriquement le pôle positif 2a des piles contenues dans le corps 1, par l'intermédiaire d'un doigt 12 en contact avec ladite rotule 7, à la tête 6 d'éclairage. Le pôle négatif 2b des piles est relié électriquement à la tête 6 d'éclairage par l'intermédiaire du corps 1, de l'écrou 8 et de la partie externe de la tige 5, ces parties étant complètement isolées du circuit électrique reliant le pôle positif 2a des piles à la tête 6 d'éclairage par une matière isolante 13.

La tête 6 comprend deux filetages 14 situés de part et d'autre de la source lumineuse 10, ces filetages 14 étant prévus pour la fixation d'au moins un miroir amovible.

Ce dispositif est construit avec des matériaux de base tels que l'aluminium et la résine époxy, afin de rendre son entretien et sa stérilisation conformes aux normes internationales.

Sur la figure 2, la tête 6 comprend un miroir 15 porté par un bras 16 vissé dans l'un des deux filetages 14 situés de part et d'autre de l'axe de symétrie de l'ampoule halogène 10 prévus à cet effet.

Sur la figure 3, on peut voir que le bras 16 portant le miroir 15 est situé à 90 ° l'un par rapport à son emplacement symétrique de telle sorte que la source lumineuse 10 soit située sur la bissectrice des axes des bras 16 pouvant porter les miroirs 15. Le miroir 15 est perpendiculaire au bras 16 qui le porte. Le miroir est plan.

Ce miroir permet d'obtenir une image droite ou gauche de la région à explorer.

Dans le cas où un effet de grossissement est désiré, on peut utiliser un miroir concave.

Le dispositif selon l'invention peut servir au dépistage des affections bucco-dentaires aussi bien en milieu hospitalier qu'en cabinet privé et en ambulatoire. Ce dispositif permet de contrôler la totalité de la cavité buccale par image directe ou indirecte et de transilluminer les dents par effet de diaphanoscopie, en rendant possible le diagnostic de certaines lésions des caries cachées ou des atteintes cryptogénique de la muqueuse buccale.

Son application médicale ne se limite pas à la sphère buccale. Le dispositif peut être utilisé dans d'autres spécialités qui peuvent avoir recours à la diaphanoscopie ou qui ont besoin d'éclairer directement ou indirectement un champ réduit. Il peut avoir sa place dans toute les trousses d'urgence. Son emploi peut aussi être recommandé lors d'enquêtes épidémiologiques (OMS).

Le dispositif trouve des applications dans d'autres domaines comme la médecine vétérinaire, la recherche scientifique ou l'industrie, dans lesquelles certaines techniques industrielles nécessitent l'éclairage de champs réduits et peu accessible.

Ses avantages sont nombreux. Il est simple, entièrement autonome et d'un maniement aisé. Grâce à son éclairage halogène puissant, il permet l'observation directe et la transillumination. Grâce à la possibilité de fixer au moins un miroir réfléchissant situé d'une part ou d'autre part de la source lumineuse, il permet en plus les observations indirectes droite ou gauche.

## Revendications

1. Dispositif portatif d'éclairage, comprenant un corps de forme allongée, prolongé par une tige, cette tige présentant à son extrémité libre une tête munie d'une source lumineuse, caractérisé en ce que la tige (5) est articulée à l'une des extrémité du corps (1), et en ce que la tête (6) comporte une source lumineuse (10) décalée par rapport à l'axe de la tige (5) et au moins un miroir (15) monté de façon amovible d'une part ou d'autre part de la source lumineuse (10).

2. Dispositif portatif d'éclairage selon la revendication 1, caractérisé en ce que la tige (5) est articulée à l'extrémité du corps (1) par une rotule (7), de telle sorte qu'elle puisse tourner autour de son axe longitudinal et être inclinée par rapport à cet axe.

3. Dispositif d'éclairage selon l'une des revendications 1 ou 2, caractérisé en ce que la tige (5) articulée peut être inclinée jusqu'à un angle d'environ 22 par rapport à l'axe longitudinal du corps (1).

4. Dispositif d'éclairage selon l'une des revendications 1 à 3, caractérisé en ce que la tige (5) articulée peut tourner autour de son axe longitudinal sur 360 _{°} .

5. Dispositif d'éclairage selon l'une des revendication 1 à 4, caractérisé en ce que la source lumineuse (10) est une ampoule halogène, alimentée par des piles (2) disposée dans ledit corps (1).

6. Dispositif d'éclairage selon l'une des revendication 1 à 5, caractérisé en ce que la tige (5) est creuse et est parcourue par un conducteur électrique (11) reliant l'un des contacts de la source lumineuse à l'un des pôles de la pile (2a), l'autre contact de la source lumineuse étant relié à l'autre pôle de la pile (2b) par le corps (1), la tige (5) et la tête (6).

7. Dispositif d'éclairage selon l'une des revendications 1 à 6, caractérisé en ce que le miroir (15) est porté par un bras (16) situé à 90 l'un par rapport à son emplacement symétrique de telle sorte que la source lumineuse (10) soit située sur la bissectrice des axes des bras (16) pouvant porter les miroirs (15).

8. Dispositif d'éclairage selon la revendication 7, caractérisé en ce que le bras (16) est vissé dans l'un des deux filetages (14) pratiqués dans la tête (6).

9. Dispositif d'éclairage selon la revendication 7 ou la revendication 8, caractérisé en ce que le miroir (15) est perpendiculaire au bras (16) qui le porte.

10. Dispositif d'éclairage selon l'une des revendications 7 à 9, caractérisé en ce que le miroir (15) est plan ou concave.
